(19) Europäisches Patentamt

European Patent Office

Office européen des brevets

(11) EP 0 567 541 B1

(12) EUROPEAN PATENT SPECIFICATION

(45) Date of publication and mention
of the grant of the patent:
**07.10.1998 Bulletin 1998/41**

(21) Application number: **92903846.1**

(22) Date of filing: **06.01.1992**

(51) Int. Cl.$^6$: **A01N 25/10**, A01N 25/34

(86) International application number:
**PCT/US92/00089**

(87) International publication number:
**WO 92/12633 (06.08.1992 Gazette 1992/21)**

(54) **CARRIER FOR ACTIVE AGENTS, AND SOLID DOSAGE FORMS PREPARED THEREWITH**

TRÄGER FÜR WIRKSTOFFE UND DAMIT HERGESTELLTE FESTE DARREICHUNGSFORMEN

VECTEUR POUR AGENTS ACTIFS UTILISE DANS LA PREPARATION DE FORMES GALENIQUE SOLIDE

(84) Designated Contracting States:
**AT BE CH DE DK ES FR GB GR IT LI LU MC NL SE**

(30) Priority: **16.01.1991 US 642099**

(43) Date of publication of application:
**03.11.1993 Bulletin 1993/44**

(73) Proprietor: **FMC CORPORATION**
**Philadelphia, PA 19103 (US)**

(72) Inventors:
• **IBRAHIM, Nagui, Iskandar**
**East Windsor, NJ 08520 (US)**
• **MEHRA, Dev, Kumar**
**Furlong, PA 18925 (US)**
• **FLECK, Edwin, George**
**Newark, DE 19711 (US)**

(74) Representative:
**Hamilton, Raymond et al**
**FMC Corporation (UK) Limited**
**Process Additives Division**
**Tenax Road**
**Trafford Park**
**Manchester M17 1WT (GB)**

(56) References cited:
**EP-A- 0 128 482        EP-A- 0 167 191**
**BE-A-   786 632         US-A- 4 681 765**

• **CENTRAL PATENTS INDEX, BASIC ABSTRACTS JOURNAL Section Ch, Week 7238, Derwent Publications Ltd., London, GB; Class C3, AN 60936T & JP-A-7 237 012 (ASAHI CHEMICAL IND CO LTD) 18 September 1972**
• **CENTRAL PATENTS INDEX, BASIC ABSTRACTS JOURNAL Section Ch, Week 8425, 15 August 1984 Derwent Publications Ltd., London, GB; Class C03, AN 155496 & JP-A-59 082 303 (SEIHO KAGAKU KOFUN YUGEN (TOKU)) 12 May 1984**

EP 0 567 541 B1

## Description

This invention relates to finely divided particulate combinations useful as carriers for active agents, which agents are intended for dispersion or dissolution in an aqueous medium.

The invention further relates to solid dosage forms produced by shaping a combination of a finely divided carrier material and an active agent, wherein the dosage form disintegrates rapidly in an aqueous medium for release therein of the active agent.

Active agents are commonly combined with particulate carriers in solid dosage forms for a variety of uses, principally for pharmaceutical and industrial applications. In many industrial applications, solid dosage forms, such as tablets, have advantages over liquid systems and particulate blends or agglomerates of active agents and particulate carriers. These include greater handling convenience and safety (respecting both humans and the environment), better control of application rate, and fewer problems in disposing of containers (because of minimal contact of active agent with the container).

Nevertheless, solid dosage forms have not been favored for application of agricultural chemicals such as herbicides and pesticides and for application of actives in certain other industrial fields because of the difficulty of identifying low cost, particulate carriers which provide good binding, compressibility (in the case of compacted forms such as tablets), and disintegration properties while also exhibiting stability during storage and transportation. More specifically, solid dosage forms for agricultural and analogous industrial applications must be strong enough to survive packaging and handling without breaking or chalking. They must also disintegrate at a desired rate in an aqueous medium, including cold water. Once dispersed in the aqueous medium (such as in the tank of a crop sprayer), the carrier material with active agent must remain in suspension, without settling, for sufficient time to permit application (if an agricultural chemical) to a crop or to the locus thereof.

Microcrystalline cellulose (MCC) is well known as a particulate carrier or excipient for pharmaceutical actives, alone or in combination with other carrier materials, in solid dosage form (tablets, pellets, beads, and the like) as illustrated in U.S. patents 4,744,987, 4,820,522, 4,517,179 and 3,951,821. MCC can also be used in granules, powders and dusts as a carrier for many other active agents, including pesticides. See U.S. patents 4,517,381, 4,816,064, 4,249,938 and Japanese patent 72/37012 granted in 1972 on an application filed June 9, 1970.

MCC is used in formed extruded foods, although these forms (such as French fries and snack foods) are not dosage forms in the usual sense, that is, are not designed for delivery of predetermined unit quantities of an active agent. See Glicksman, Food Hydrocolloids, Vol. III, 1986, CRC Press, Inc., 34-42.

As indicated in U.S. patent 4,744,987, MCC has excellent binding and compressibility characteristics for solid dosage form manufacture and these compacts disintegrate rapidly in aqueous media. Unfortunately, MCC in large proportions is often too expensive as a carrier for active agents other than pharmaceuticals, particularly active agents which are applied over extensive areas, as is the case with agricultural chemicals such as herbicides and pesticides. It is therefore desirable to dilute the MCC with a co-carrier in order to reduce cost, but without unduly compromising the benefits of the MCC. However, low cost co-carriers must not inhibit the compression properties as well as the rapid disintegration of the compact at the lower temperatures often encountered in the application of agricultural chemicals or which may be a circumstance in other uses.

Urea is known as a carrier for herbicides, as described in U.S. patents 4,517,381, 4,816,064 and 4,249,938. Although these patents also mention MCC, there is no teaching or suggestion therein that these materials may be used in combination as carriers in solid dosage forms. Urea, of course, is well known as a plant food and fertilizer, and as a feed supplement for ruminants. It is also used in pharmaceutical preparations as a diuretic and for lowering intercranial pressure.

Urea alone is hygroscopic and therefore tends to cake very easily, producing hard masses which cannot be distributed efficiently and uniformly over soil to be fertilized. Such masses also tend to dissolve slowly. In response to this problem, U.S. patent 3,558,299 discloses a process for preparing noncaking, particulate urea by coating the urea with a mixture of an inert substance and urease. Inert substances disclosed are finely divided clay (talc, mica) or a powdered organic material such as nutshells or pulverized corncobs. MCC or solid dosage forms, such as granules or tablets, are not mentioned.

Japanese unexamined patent application 59/82303, filed November 2, 1982 and published May 12, 1984, describes an agricultural chemical composition which readily disintegrates in water. The composition comprises a wettable powder of a) an agricultural chemical (fertilizer, herbicide, et cetera), b) an inorganic powder (calcium silicate), and c) a fine powdered resin which swells when water is absorbed (graft copolymer of cellulose, polyacrylate, et cetera). Urea, MCC and/or solid dosage forms apparently are not disclosed.

In accordance with the present invention a finely divided particulate combination of MCC and at least one nitrogen-containing compound, which is urea, ammonium sulphate, ammonium phosphate or a mixture thereof, which is sufficiently water soluble or water swellable so that it readily disperses in an aqueous medium, in an MCC:nitrogen-containing compound weight ratio in the range of about 10:1 to 1:10, provides a carrier which can be admixed with an active agent and shaped to produce an effective solid dosage form.

The carrier may further include a hydrocolloid, such as sodium carboxymethyl cellulose (NaCMC), to assist in suspension of the MCC when solid dosage forms of the invention are dispersed in aqueous media.

This invention provides solids dosage form which comprises an active agent and a carrier which disintegrates rapidly in an aqueous medium to release the active agent, characterised in that the carrier comprises:

a) microcrystalline cellulose;

b) at least one nitrogen-containing compound which is: urea, ammonium sulfate; ammonium phosphate or a mixture thereof, the nitrogen containing compound being different from the active agent, the ratio by weight of microcrystalline cellulose to nitrogen-containing compound being in the range of 10:1 to 1:10; and

c) optionally, a minor amount of one or more suspending agents for the microcrystalline cellulose selected from water-soluble hydrocolloids compatible with and effective for assisting in the hydration of the microcrystalline cellulose.

The solid dosage forms of the invention are sufficiently strong to survive packaging and handling without breaking or chalking. Moreover, the solid dosage forms disintegrate rapidly in an aqueous medium, even at low temperatures, and the disintegrated, suspended particles will not clump or pack when left undisturbed for extended periods, of the order of several days longer. when the active agent is a material which may be toxic when handled in the isolated state, such as a herbicide or pesticide, the solid dosage form improves handling and application safety.

By virtue of the invention the benefits of MCC in solid dosage forms are now made available to delivery of active agents in applications where carrier cost is a significant factor. This is achieved along with surprisingly improved properties, including rapid disintegration, and no diminution of properties attributable to the MCC. The property of rapid disintegration even at low ambient temperature (such as the dew point in agricultural environments) reduces avian toxicity when the active agent is a type which would present such risk if broadcast in slow disintegrating granules or extrudate form. In addition, because the carrying capacity of the composite carrier of the invention is very high, as much as 65% by weight or more of active agent in some cases, the cost effectiveness of the composition is further enhanced.

Microcrystalline cellulose (MCC) is a purified, partially depolymerized, natural cellulose used primarily as an excipient for pharmaceuticals. Its popularity is due to its outstanding compressibility characteristics, resulting in good binding and disintegration properties, as compared with other tablet excipients such as partially pregelatinized cornstarch, lactose, and dicalcium phosphate. However, MCC is relatively costly because of the steps required in its manufacture. This tends to limit its usefulness in price-sensitive markets such as agricultural chemicals.

In accordance with the present invention the cost of MCC is offset by combining MCC with one or more water-soluble or water swellable, nitrogen-containing neutral compounds, which are compatible with and different from the active agent, to produce a particulate mixture. The active agent is then admixed with the particulate carrier mixture and the combination is shaped by conventional means into a solid dosage form. The resulting shaped masses may vary widely in shape and size. They may have a regular or irregular appearance, and may be rounded, disk-shaped, flat, or oblong. Suitable forms are variously described as tablets, pellets, beads, balls, cakes, compacts, granules, bars, briquettes and the like. The forms may not only be compression molded but may also be extruded, pelletized, granulated, die, cast, briquetted or shaped in any other manner.

MCC materials useful in the present invention are powdered substances sold under "Avicel", "Lattice" and other trademarks. The carrier particle size for optimum results depends on the manner in which the MCC is combined with the co-carrier, the disintegration rate desired for solid dosage forms prepared with the MCC/co-carrier mixture, and the stability of aqueous suspensions produced upon disintegration of the solid dosage forms. For example, if the combination of carrier and active agent is to be compressed as a dry blend in the tabletting machine, smaller size particles will flow poorly in the machine and the hopper or feed mechanism will tend to clog. However, small particles (average size about 10-20 microns) will provide a more uniform and stable suspension upon disintegration of the tablets in an aqueous medium. Conversely, large particles (average size about 90 microns and larger) will flow better in the tabletting machine but will not stay in suspension upon disintegration of the tablets.

Accordingly, when the composition is to be shaped by compression of a dry blend, it has been determined that effective carrier particle sizes are governed primarily by the MCC particle size. Useful MCC average particle sizes range from about 20 to 90 microns, most preferred about 50 microns.

The water soluble or swellable nitrogen-containing co-carrier is selected from the group comprising urea, ammonium sulphate, ammonium phosphate and mixtures thereof, and is neutral and stable in the carrier system of the invention, that is, it will not hydrolyze or otherwise decompose. The co-carrier should also be compatible, that is, will not chemically interact, with the active agent, and otherwise be effective for the desired application of the solid dosage form. While the co-carrier acts as an extender for the MCC in order to reduce the cost of total carrier composition, it also exhibits a "wicking" action and thereby promotes disintegration of the carrier composition in water. This increase the rate of disintegration beyond that attributable to MCC alone and improves the suspension characteristics, upon disintegration of the finished dosage form.

This invention also provides a method for lowering the disintegration time in water of a compacted composition comprising an active ingredient and microcrystalline cellulose characterised in that a nitrogen containing compound selected from the group comprising urea, ammonium sulphate and ammonium phosphate is incorporated into the composition in an amount such that the ratio of the weight of microcrystalline cellulose to nitrogen containing compound is in the range 10:1 to 1:10.

The rate of disintegration can be slowed or otherwise controlled to a desire degree by increasing the proportion of MCC in the carrier mixture and also by adding a gelling agent to the mixture. Suitable gelling agents are well-known and include polyacrylamide and other gel forming hydrocolloids.

Disintegration of compact and the subsequent release rate of the active agent are primarily controlled by the aqueous solubility of the active, but are also related to the amount of active in the dosage form. Generally, the higher the loading of the active the slower the disintegration and release rate. Effective disintegration and release rates for specific active agents are determinable by routine testing and trial.

Suitable nitrogen-containing compounds are those which have sufficient surface affinity for the MCC particles to promote adherence to the MCC particles (by hydrogen bonding or other force) when subjected to a shaping force such as compression, but which also solvate at lower energy than is required for association with the MCC particles after disintegration of the solid dosage form in an aqueous medium. Under these conditions the MCC particles initially, and tightly, bind the active agent but quickly wicks in water and disaggregate to create an enormous surface area and release the active agent when the solid dosage form is added to an aqueous medium.

The nitrogen-containing compounds effective in the foregoing manner are ureas ammonium sulphate and ammonium phosphate.

The nitrogen-containing compounds may be used singly or as mixtures of two or more thereof. The selection of nitrogen-containing compound(s) is dictated not only by efficacy as a co-carrier but also by cost.

If the nitrogen-containing compound is to be dry-blended with the MCC to form a carrier composition suitable for admixture with an active agent, it should have a small particle size and flow freely for homogeneous blending with the MCC. Where urea is the co-carrier, standard industrial urea is suitable, such as urea ground to 2.36 mm - 2.0 mm sieve (8-10) USS mesh), a coarse material, or 1.40 mm - 600μm sieve (14-30 USS mesh), a finer material. Small particle size ( of the order of about 10-200 microns) is achieved by well-known techniques including crystallization, freeze-drying, a various milling processes, both of the individual components of the carrier and of the carrier mixture after dry blending. If the composite carrier is wet granulated with the active agent to form the finished dosage form, the nitrogen-containing co-carrier will dissolve into the MCC. In this case, particle size of the co-carrier is not important.

The ratio of MCC to nitrogen-containing compound in the carrier, along with carrier particle size, influences the hardness (as measured by tensile strength) of solid dosage forms prepared with the carrier, and the hardness in turn affects the disintegration rate of the forms. For example, tablets should be hard enough to resist chalking and breaking during normal handling but readily disintegrate in an aqueous medium. Tablets with a tensile strength in the range of about 2-25 kg/cm$^2$ will resist breaking during normal handling and will disintegrate quicky in an aqueous medium. A preferred tensile strength is about 3-15 kg/cm$^2$, most preferred about 3-10 kg/cm$^2$. Suitable MCC:nitrogen-containing compound ratios for such purposes are from about 10:1 to about 1:10 by weight, preferably about 5:1 to about 1:5, most preferably about 1:1.

The components of the carrier composition are combined in any suitable manner to produce a dry, finely divided, particulate blend or powder. Techniques for producing such blends or powders are well known and include dry blending or granulation, wet granulation followed by oven drying, and aqueous dispersion followed by spray drying. Dosage forms may be produced from the carrier composition and active agent by any of the known processes, including direct compression and/or roller compaction of dry blends, wet granulation followed by compaction, and/or spheronization.

When tabletting is the method selected for producing the solid dosage forms, conventional dry granulation, wet granulation, direct compression, spheronization or spray drying may be used to prepare the carrier composition for the tabletting. The selection of method depends primarily on the active agent, the ability of the mixture of carrier and active to flow freely in the tabletting machine or extruder, and the cohesiveness of the ingredients. If the active agent can be admixed with the carrier to produce a free flowing, dense powder, the mixture can be directly compressed. In dry granulation, a dry powdery blend of the carrier components and active agent is compressed to form slugs if a tablet press is used. Alternatively, the dry blend is roller compacted into sheets. The slugs or sheets are then sieved (for example, to 1.70 mm, 1.40 mm or 1.18 mm sieve [to 12, 14 or 16 USS mesh]) to form densified granules for final tabletting.

In one mode of wet granulation, water is added to a mixture of the MCC and co-carrier to form a wet, granular material. The amount of water depends on the ratio of MCC to co-carrier. The higher the MCC:co-carrier ratio by weight, the greater will be the amount of water which can be tolerated. Generally, about one-third of the mixture of MCC, co-carrier and water will be water when the MCC:co-carrier ratio is about 1:1 by weight. The wet, granular material is screened (to 2.00 mm sieve [10 USS mesh], for example), dried, and again screened (to 850 μm sieve [20 USS mesh], for example). The granules are then ready for admixture with the active agent and other tabletting ingredients, if any.

In a variation of the wet granulation process, the MCC may be "coprocessed" with the nitrogen-containing co-carrier in the manner described in U.S. patent 4,744,987 where the nitrogen-containing compound is substituted for the calcium carbonate of the patent. In "coprocessing", a uniform aqueous slurry of the MCC and co-carrier is formed with good agitation and the slurry is dried in any effective manner. Spray drying is preferred. MCC:co-carrier ratios may be the same as in other blending methods and the particulate product may be screened as needed to obtain a desired particle size distribution. The coprocessed carrier material may then be combined with an active agent in the same manner as described above with respect to dry or wet granulation and direct compression.

Wet granulation or extrusion can be used to prepare carrier composition for all active agents except those which are sensitive to water, that is, actives which hydrolyze or otherwise decompose when admixed with the carrier.

In accordance with another aspect of this invention, the MCC can be prevented from settling out when solid dosage forms of the invention are dispersed in aqueous media by incorporating into the carrier composition a minor amount of a hydrocolloid compatible with the MCC and effective for assisting in wetting out (hydrating) the MCC. Such agents thus act to suspend the MCC and are further characterized by water solubility and by bonding or otherwise adhering to the MCC. They may be natural or synthetic and generally are polymeric carbohydrates.

Typical of the suspending agents are cellulose derivatives such as sodium carboxymethyl cellulose, hydroxyethyl cellulose, hydroxypropyl cellulose, hydroxypropylmethyl cellulose and methyl cellulose; gums such as seaweed extracts (agar, carrageenan, alginates), plant extracts (pectins, low methoxy pectins), plant exudates (arabic, tragacanth, karaya, ghatti), plant seed extracts (locust bean, guar), and microbial fermentation gums (dextran, xanthan); starches including pregelatinized and modified starches; and synthetics such as the carboxyvinyl polymers sold under the brand name "Carbopol".

The preferred suspending agents are salts such as sodium carboxymethyl cellulose. While not fully understood, it is believed that best suspending action results from particle repulsion forces due to the ionic character of such materials.

The suspending agents are used in small amounts, of the order of about 0.05-25% by weight of the MCC, preferably about 0.5-10% by weight, taking into account the molecular weight and correlative viscosity of the agents. In the case of the preferred agent, sodium carboxymethyl cellulose, medium or high viscosity materials are preferred due to the higher rate of hydration of these materials, although the lower viscosity materials will disperse more readily.

The suspending agent may be admixed with the MCC and co-carrier in any effective manner and the order of addition is not critical. If the carrier composition is prepared from aqueous dispersion, for example, by wet granulation or spray drying, the suspending agent is usually added after admixture of the MCC and co-carrier in the aqueous medium, with agitation effective for uniform admixture.

Various ingredients well-known to formulators of solid dosage forms can be added to carrier compositions of the invention to enhance the blending, shaping and/or disintegration processes. These include lubricants, glidants, dispersants, surfactants, disintegrants, and fillers or auxiliary binders, and are employed in relatively small amounts, less than about 20% by weight of the solid dosage form, more usually less than about 10% by weight. The choice of additive and amount are matters of routine consideration and trial for the skilled formulator.

Lubricants facilitate the ejection of compacted forms from a die cavity. They may also reduce interparticle friction (glidant functionality) and prevent adhesion of materials to die and punch surfaces (anti-adherent properties). Typical lubricants are talc; long chain fatty acid esters or salts thereof such as stearic and palmitic acids, and magnesium or calcium stearate; and other materials such as hydrogenated vegetable oils, glyceryl palmitostearate, gyceryl benzoate, and polyethylene glycol 6000. Glidants are limited to improvement of the flow properties of powders and granules, and include materials such as aerogenic silica, fumed silicon dioxide and silica hydrogel. Typical use levels of lubricants and glidants are about 0.25-5 wt % of each, based on total dosage form weight.

Auxiliary disintegrants may be added to the carrier compositions to increase the breakup rate of the solid dosage forms in an aqueous medium, particularly at low temperatures. Typical disintegrants are starch (corn, potato, amylose), sodium starch glycolate, alginic acid, synthetic polymers such as styrene-divinylbenzene or acrylic ion exchange or adsorbent resins, crosspovidone, and soy polysaccharides. A preferred disintegrant is croscarmellose, a cross-linked sodium carboxymethyl cellulose sold under the trademark "Ac-Di-Sol". The disintegrants typically are used at levels of about 1-5 wt % based on total dosage form weight.

The active agents to be delivered with the solid carrier of the invention include any liquid or solid compounds or mixtures of compounds which are to be eventually dispersed or dissolved in an aqueous medium either to prepare a secondary carrier (for example, a solution or suspension to be sprayed) or to operate directly at the use site (for example, an industrial process or waste stream, a body of water such as a river or lake, a fluid bed, a swimming pool, an oil well, a body fluid, an aqueous food, food supplement or pharmaceutical, and the like). Of course, the active agent must not be reactive with the carrier components in a manner that reduces or interferes with the functions of the carrier. The carrier and solid dosage form of the invention therefore have application to a wide variety of fields and products, including agricultural and veterinary products, pharmaceuticals, animal and human foods, swimming pool additives, industrial biocides for oil wells and other applications, cosmetics, household pesticides, and dye manufacturing.

In the agricultural field the active agents include herbicides, plant growth regulators, and biocides of all types, such as pesticides. The pesticides include atrazine, bentazone, trifluralin, propanil, metribuzin, alachor, butachlor, bromoxynil, clomazone, oxadiazon, lorsban, bifenox, aldicarb, monocrotophos, propoxur, diflubenzuron, carbofuran, permethrin, carbaryl, cypermethrin, endosulfan, cyfluthrin, bifenthrin, terbufos, fenamiphos, cadusafos, paclobutrazol, glyphosine, giberellic acid, and the like.

Shaping processes in addition to tabletting can be used to prepare solid dosage forms of the invention, and techniques and equipment for such purposes are well-known. Such processes include pelletizing, extrusion, agglomeration and spheronization, and are extensively treated in textbooks and other publications on the subject.

The carrier compositions of the invention permit great flexibility in dosage form design. For example, compressed forms such as tablets can be produced in bisected or multisected form so that they can be subdivided by hand or machine for delivery of fractional amounts of active agent. Such tablets are particularly useful for delivery of pesticides, herbicides and other agricultural chemicals for home and garden, green house, forestry, and farm markets without significantly compromising bioefficacy. Furthermore, chemically incompatible actives, such as tablets containing several different pesticides or combinations of herbicides and pesticides, can be separated in the solid dosage forms by forming multilayers using known techniques.

The following examples are given to further illustrate the invention. As used herein the sieve sizes are designated in ASTM standard E11-87.

## EXAMPLE 1

## DRY-BLEND PROCESS

AVICEL® PH 105 microcrystalline cellulose (MCC, 20 microns average particle size, FMC Corporation) and urea granules (milled to 850 $\mu$m - 250 $\mu$m sieve, [20-60 USS mesh]) were dry-blended in a PK mixer to form five blends of different proportions (Table 1). Four grams of each blend was used to prepare 2.54 cm diameter tablets in a Carver tablet press operated at 923 kg/cm$^2$ compression force, 3% moisture content. Each tablet was measured for thickness. The disintegration time was determined by immersing each tablet into 400 mL of room temperature tap water. The results are presented in Table 1. It can be seen that as compared with the controls, disintegration times were substantially reduced by the presence of urea.

Table 1

| Tablet composition % wt/wt MCC/urea | Thickness of Tablet (mm) | Disintegration time[a] (sec) |
|---|---|---|
| 100/0 (control) | 6.44 | 240 |
| 66.6/33.4 | 7.32 | 90 |
| 50/50 | 6.82 | 15 |
| 25/75 | 6.41 | 30 |
| 0/100 (control) | 6.32 | no disintegration |

a. Disintegration time was measured from when the tablet began to disintegrate to when the tablet had fully disintegrated.

## EXAMPLE 2

## WET GRANULATION PROCESS

Urea (particle size same as Example 1) and AVICEL® PH 105 microcrystalline cellulose (MCC, 150 grams) were mixed in a Hobart mixer for five minutes to form a 1:1 urea/MCC blend. Approximately 150 mL of tap water was added during mixing, forming a wet, granular material. This material was screened using a 2.0 mm sieve (10 USS mesh). The resulting granular material was dried in an oven at 50°C until a moisture level of 3% was obtained. The dry, granular material was screened using a 850 $\mu$m sieve (20 USS mesh). These granules were used to prepare 0.5 gram tablets using a Stokes B2 model tablet press and 1.11 cm (7/16 inch) standard concave punches. Tablet hardness was measured on a Schleuniger Hardness Tester. Tablet disintegration time was determined by immersing each tablet in approximately 400 mL of water at room temperature. The results are shown in Table 2 from which it will be seen that the harder tablets (tensile 10.5 kg/cm$^2$) nevertheless disintegrate in the same time as the softer tablets (tensile 6 kg/cm$^2$), but that increasing the hardness substantially retards or prevents disintegration.

Table 2

| Tablet Compression Force (kg) | Tablet tensile[a] Strength (kg/cm$^2$ | Disintegration[b] Time (sec) |
|---|---|---|
| 190 | 6 | 10 |
| 380 | 10.5 | 10 |
| 700 | 31 | no disintegration[c] |
| 1050 | 35 | no disintegration[c] |

a. Tablet tensile strength was calculated according to the following formula:
$TS = {}^{2H}/\pi DT$, in which H=tablet hardness (Kg), D=tablet diameter (cm), and
T=tablet thickness (cm)
b. Disintegration time was measured from the moment the tablet was immersed in
the water until it had fully disintegrated.
c. Tablet did not disintegrate within five minutes.

EXAMPLE 3

HERBICIDE TABLETS PREPARED BY DRY GRANULATION

A mixture of 4.15 kg of atrazine herbicide, 45.4 g of AC-DI-SOL® croscarmellose disintegrant (FMC Corporation), 226.8 g of a sodium lignosulfonate anionic dispersant, 45.4 g of an alkylarylsulfonate surfactant, and 68.0 g of an aerogenic synthetic silica were mixed in a PK blender for ten minutes. The resultant blend was milled in a Fitz mill at a rate of one pound per minute using a 0.05 cm (0.02 inch) opening screen. A portion of the milled material (1.34 kg) was transferred to a blender, and 228.0 g of of AVICEL® PH 101 microcrystalline cellulose (50 microns average particle size, FMC Corporation), 571.5 grams of urea (same as in Example 1), and 22.7 of AC-DI-SOL® croscarmellose disintegrant were added. The resultant mixture was blended for five minutes. Talc (40.4 g) and magnesium stearate (7.48 g) were added as lubricants and the mixture was blended for five minutes. This mixture was slugged using a vibratory screw feeder which fed directly into the feed frame of a Colton 250 tablet press using a 4.6 cm (1 and 13/16 inch) flat face beveled edge punch. The slugged material was crushed in a Fitz mill fitted with a 1.70 mm sieve (12 USS mesh), providing a granular material. These granules were compressed on a Stokes model R tablet press set at a rate of 16 tablets per minute. The tablet diameter was 6.67 cm (2 5/8 inches).

The 60 g tablets which were produced remained intact when dropped from a height of four feet to a hard surface indicating excellent hardness. The tablets disintegrated completely within 2 to 2.25 minutes when immersed in four liters of tap water at room temperature, demonstrating a disintegration rate acceptable for preparation of herbicide suspensions for spray application.

EXAMPLES 4-18

Other herbicide tablets were prepared essentially as described in Example 3 to determine the effects on hardness and disintegration time of compression force and of various proportions of MCC, urea and various additives in the carrier. The data (Table 3) shows that both hard (high tensile strength) and soft (lower tensile strength) tablets can be produced with carrier compositions of the invention but having disintegration (dissolving) times of similar magnitude, by varying the proportions of MCC and urea and by changing the amounts of lubricant and/or glidant (compare Ex. 4, 6, 7, 9, and 10). Examples 10-14 show the effect of varying the compression force, in preparing the tablets, on disintegration times. It will be seen that lower hardness (tensile strength) leads to shorter disintegration times, and vice versa.

Table 3
ATRAZINE:MCC:UREA TABLET FORMULATIONS
DRY-BLEND PROCESS
(% by weight)

| Example No. | Atrazine | MCC | Urea | Disintegrant[g] | Lubricant[a] | Glidant[b] | Surfactant[c] | Dispersant[d] | Tablet Tensile Strength[e] | Time[f] (sec) |
|---|---|---|---|---|---|---|---|---|---|---|
| 4 | 31.3 | 40.0 | 21.4 | 3.0 | 2.3[1] | 2.0[1] | - | - | 12.5 | 75 |
| 5 | 31.25 | 10.0 | 53.15 | 2.0 | 2.6[2] | 1.0[2] | - | - | 12.5 | 120 |
| 6 | 31.25 | 10.0 | 53.15 | 2.0 | 2.6[2] | 1.0[2] | - | - | 5.5 | 90 |
| 7 | 62.5 | 10.0 | 18.9 | 3.0 | 2.6[2] | 3.0[1] | - | - | 5.0 | 210 |
| 8 | 62.5 | 10.0 | 19.4 | 3.0 | 2.6[2] | 2.5[2] | - | - | 3.0 | 180 |
| 9 | 62.5 | 10.0 | 20.9 | 2.0 | 0.6[3] | 4.0[3] | - | - | 3.0 | 90 |
| 10 | 52.7 | 10.0 | 25.7 | 2.0 | 0.6[3] | 3.0[2] | 1.0 | 5.0 | 13.0 | 75 |
| 11 | 52.7 | 10.0 | 25.7 | 2.0 | 0.6[3] | 3.0[2] | 1.0 | 5.0 | 11.0 | 120 |
| 12 | 52.7 | 10.0 | 25.7 | 2.0 | 0.6[3] | 3.0[2] | 1.0 | 5.0 | 5.5 | 45 |
| 13 | 52.7 | 10.0 | 25.7 | 2.0 | 0.6[3] | 3.0[2] | 1.0 | 5.0 | 4.0 | 30 |
| 14 | 52.7 | 10.0 | 25.7 | 2.0 | 0.6[3] | 3.0[2] | 1.0 | 5.0 | 2.5 | 30 |
| 15 | 52.8 | 13.96 | 19.9 | 2.66 | 0.6[3] | 3.48[2] | 1.32 | 5.28 | | >120 |
| 16 | 52.7 | 10.0 | 25.7 | 2.0 | 0.6[3] | 3.0[2] | 1.0 | 5.0 | 1.5 | >120 |
| 17 | 51.85 | 9.84 | 25.29 | 2.97 | 1.2[4] | 2.95[1] | 0.98 | 4.92 | 6.0 | >120 |
| 18 | 54.46 | 12.7 | 24.0 | 1.41 | 2.3[1] | 1.59[1] | 0.59 | 2.95 | | 240 |

EP 0 567 541 B1

a.    Lubricants consisted of talc and/or magnesium stearate.
1 = 2.0% talc/0.3% Mg stearate
2 = 2.0% talc/0.6% Mg stearate
3 = Mg stearate only
4 = 1.0% talc/0.2% Mg stearate

b.    Glidants (synthetic silicas)
1 = aerogenic silica
2 = fumed silica
3 = 3.0% aerogenic silica/1% fumed silica

c.    Surfactant used was an alkylarylsulfonate

d.    Dispersant used was a sodium lignosulfonate

e.    Calculated as in Example 2.

f.    Measured as in Example 2.

g.    AC-DI-SOL® croscarmellose

EXAMPLE 19

Pharmaceutically Active Spheres prepared by Extrusion/Spheronization

Fast disintegrating spheres of hydrochlorothiazide (active) were prepared from the following formulation:

| Ingredients | wt % | wt/wt |
|---|---|---|
| 1) Hydrochlorothiazide | 16.00 | 80 |
| 2) MCC/Urea (1:2) | 23.52 | |
| 3) Polyfon H surfactant | 0.24 | 20 |
| 4) Ac-Di-Sol croscarmellose | 0.24 | |
| 5) Dicalcium Phosphate | 57.00 | 285 |
| 6) Celatom FP4 diatomite | 2.00 | 10 |
| 7) Ac-Di-Sol croscarmellose | 1.00 | 5 |

(continued)

| Ingredients | wt % | wt/wt |
|---|---|---|
| | 100.00 | 500 g |

The MCC/urea ingredient was a coprocessed material prepared by dissolving urea in water using a dispersator mixer, adding MCC in wetcake form to provide an MCC/urea weight ratio of 1:2, dispersing the MCC in the solution, and spray drying the mixture to form a powder having 2% (wt.) moisture content.

The Polyfon H surfactant (Westvaco) is a sodium lignosulfonate anionic dispersant. The Celatom FP4 diatomite is diatomaceous earth (Eagle-Picher). Ingredients 1-5 were blended in 2 quart Hobart mixer for 2 minutes at speed setting 1. Water, 140 ml, was added incrementally over a 3 minute period while mixing at speed setting 1. The wet mass was blended an extra 1 minute at speed setting 1 and then forced by hand through a 1.18 mm sieve (16 USS mesh) and spheronized. The spheres were dried in a tray oven at 50°C to 1% moisture level.

EXAMPLE 20

WET GRANULATION PROCESS - MCC/AMMONIUM SULFATE

Avicel$^®$ PH105 microcrystalline cellulose (MCC), 500 grams, and 320 grams of ammonium sulfate were mixed in a Hobart mixer at a slow speed for five minutes. While still mixing at a slow speed, a solution of 180 grams of ammonium sulfate in 375 grams of water was added portionwise during a one minute period. Care was taken to prevent solid build-up on the sides and paddle of the mixer. A wet, granular material that was a 1:1 blend of MCC/ammonium sulfate was formed. This material was screened using a 2.0 mm sieve (10 USS mesh). The screened material was then placed on trays and dried in a convection oven at 50°C for six hours to bring the moisture content to 2-3%. The dry, granular material was screened through a 850 μm sieve (20 USS mesh). For those tablets containing a lubricant, the dry, screened granules were first placed in a "v" blender and blended for five minutes with about 10 grams (1% wt/wt) of magnesium stearate as lubricant. Similar tablets combining urea and the sulfate as co-carriers were also prepared.

The dry, granular material, optionally containing the lubricant, was used to prepare 500 mg tablets using a Stokes model B2 tablet press tooled with 1.11 cm (7/16 inch) standard concave punches. Tablet hardness was measured on a Schleuniger Hardness Tester. Tablet disintegration time was determined by immersing each tablet in approximately 700 mL of water at room temperature using a USP disintegration basket apparatus. Test results are given in Table 4 below in comparison with urea as co-carrier. The good compressibility and disintegration times for tablets prepared with the MCC/ammonium sulfate composition indicate that the composition is an effective excipient for tabletting of active agents such as agricultural chemicals.

EXAMPLE 21

WET GRANULATION PROCESS-MCC/AMMONIUM PHOSPHATE

Avicel$^®$ PH101 microcrystalline cellulose, 500 grams, was screened through a 425 μm sieve (40 USS mesh). The screened MCC and 300 grams of ammonium phosphate were blended at slow speed in a Hobart mixer. With continued blending, a solution of 200 grams of ammonium phosphate in 500 ml of water was added during a two minute period. After this time blending was continued for an additional two minutes. A wet, granular material that was a 1:1 blend of MCC/ammonium phosphate was formed. This material was screened using a 1.70 mm sieve (12 USS mesh). The screened material was then placed on trays and dried in a convection oven at 50°C for six hours to bring the moisture content to 2-3%. Addition of lubricant and the tabletting process were conducted essentially as described in Example 20. Disintegration time was not determined by USP methods, but rather by visual observation of the tablets when they were placed in 250 ml of water. Test results are set forth in Table 4 from which it can be concluded that the MCC/ammonium phosphate composition is an effective excipient for tabletting of active agents such as agricultural chemicals.

## Table 4
### Test Results on Examples 20 and 21

| MCC[1] | Tablet Composition (% wt/wt)[3] | | | | Tablet Tensile Strength (Kg/Cm$^2$) | Compression Force (Kg) | Ejection Force (Kg) | Disintegration Time (Seconds) |
|---|---|---|---|---|---|---|---|---|
| | UREA | $(NH_4)_2SO_4$ | $(NH_4)_3PO_4$ | | | | | |
| 50 | 50 | - | - | Unlubricated | 6 | 202 | 3 | 8.9 |
| | | | | | 9 | 317 | 10 | 10.8 |
| | | | | | 12 | 426 | 18 | 11.5 |
| | | | | | 18 | 640 | 23 | 108.0 |
| | | | | Lubricated[2] | 5 | 198 | 4 | |
| | | | | | 9 | 333 | 5 | |
| | | | | | 13 | 508 | 5 | |
| | | | | | 18 | 808 | 5 | |
| 50 | 25 | 25 | - | Unlubricated | 4 | 213 | 17 | 7.0 |
| | | | | | 8 | 392 | 25 | 15.7 |
| | | | | | 12 | 517 | 28 | 32.0 |
| | | | | | 18 | 844 | 28 | 470.0 |
| | | | | Lubricated | 4 | 349 | 5 | |
| | | | | | 9 | 609 | 6 | |
| | | | | | 13 | 935 | 5 | |
| | | | | | 15 | 1460 | 4 | |
| 50 | - | 50 | - | Unlubricated | 4 | 195 | 23 | 24.0 |
| | | | | | 8 | 355 | 33 | 104.0 |
| | | | | | 12 | 512 | 35 | 147.0 |
| | | | | | 15 | 654 | 40 | 513.0 |
| | | | | Lubricated | 4 | 409 | 6 | |
| | | | | | 8 | 662 | 8 | |
| | | | | | 12 | 918 | 9 | |
| | | | | | 15 | - | - | |
| 25 | - | 75 | - | Unlubricated | 4 | 412 | - | 24.0 |
| | | | | | 8 | 686 | 35 | 27.0 |
| | | | | | 13 | 1031 | 57 | 29.0 |
| | | | | | 17 | 1287 | 64 | 72.0 |
| | | | | Lubricated | 4 | 675 | 1 | |
| | | | | | 8 | 1012 | 2 | |
| | | | | | 12 | 1556 | 2 | |
| | | | | | 16 | 1291 | 2 | |

EP 0 567 541 B1

| MCC[1] | Tablet Composition (% wt/wt)[3] UREA | (NH$_4$)$_2$SO$_4$ | (NH$_4$)$_3$PO$_4$ | | Tablet Tensile Strength (Kg/Cm$^2$) | Compression Force (Kg) | Ejection Force (Kg) | Disintegration Time (Seconds) |
|---|---|---|---|---|---|---|---|---|
| | | | | Lubricated | 4 | 675 | 1 | |
| | | | | | 8 | 1012 | 2 | |
| | | | | | 12 | 1556 | 2 | |
| | | | | | 16 | 1291 | 2 | |
| 25 | 37.5 | 37.5 | - | Unlubricated | 5 | 293 | 14 | 516.0 |
| | | | | | 8 | 534 | 20 | 1025.0 |
| | | | | | 13 | 593 | 40 | 41.0 |
| | | | | | 16 | 763 | 48 | 51.0 |
| | | | | Lubricated | 4 | 375 | 6 | |
| | | | | | 8 | 646 | 9 | |
| | | | | | 12 | 945 | 12 | |
| | | | | | 15 | 3620 | 27 | |
| 25 | 75 | - | - | Unlubricated | 4 | 309 | 12 | 8.0 |
| | | | | | 8 | 485 | 18 | 16.0 |
| | | | | | 12 | 723 | 23 | 19.0 |
| | | | | | 17 | 1115 | 24 | 67.0 |
| | | | | Lubricated | 4 | 608 | 5 | |
| | | | | | 8 | 1691 | 7 | |
| | | | | | 8 | 4927 | 9 | |
| 50 | - | - | 50 | Unlubricated | 4 | 350 | 23 | |
| | | | | | 6 | 487 | 58 | |
| | | | | | 12 | 734 | 78 | $\leq 60$[4] |
| | | | | | 23 | 1273 | 97 | |
| | | | | Lubricated | 4 | 408 | 6 | |
| | | | | | 9 | 711 | 8 | |
| | | | | | 15 | 1033 | 9 | |
| | | | | | 23 | 1584 | 10 | |

(1)  Avicel$^R$ PH105 microcrystalline cellulose (MCC) was used in MCC/UREA/(NH$_4$)$_2$SO$_4$ Tabletting.  Avicel$^R$ PH101 microcrystalline cellulose was used in the MCC/(NH$_4$)$_3$PO$_4$ tabletting.

(2)  Tablets were lubricated with 1% (wt/wt) magnesium stearate.

(3)  Tablets of 500 mg were prepared.

(4)  Disintegration time was not determined by USP methods, but rather visually in 250 ml of water.

EXAMPLE 22

CARRIER COMPOSITIONS WITH A SUSPENDING AID

In a typical run, a slurry was prepared by combining, sequentially, 80 gallons of distilled water, 46.77 kg (102 pounds) of urea, 2.1 pounds of a food grade CMC (viscosity = 25-50 centipose at 2% concentration; degree of substitution = 0.7) and 115.2 kg (254 pounds) of attrited MCC filtered wet cake (39% MCC in water). The slurry was passed once through an in-line colloid mill to fully disperse the solids prior to spray drying. The spray drier conditions were adjusted to deliver a final particle size of about 30 to 50 $\mu$m and a water content of about 2-3%. The resultant powder was tabletted by the wet granulation method and disintegration time was determined, both as described in Example 2. A disintegration time of 5 minutes or less is considered optimum. Suspension numbers were determined by the CIPAC method MT-15 where the higher number indicates improved suspension. Tablets of MCC/urea without a suspending aid settle immediately upon disintegration in water and have a suspension number = 0.

Test results are given in Table 5. It will be seen that both urea and CMC were necessary for good suspension and that the best formulation overall (fast disintegration and good suspension) is formulation 3.

EP 0 567 541 B1

Table 5
Characteristics of Tablets Containing MCC, CMC and Urea

| Form- ulation | Composition (% wt/wt) | | | Tablet Hardness (Kg) | Disintegration Time of Tablet (min.) | Suspension Number | Percent Residue On 150 μm sieve (100 USS Mesh Screen) |
|---|---|---|---|---|---|---|---|
| | MCC | CMC | UREA | | | | |
| 1 | 99.0[1] | 1.0 | - | Powder | | 12.2 | - |
| | | | | 4 | <0.5 | 3.9 | 5.9 |
| | | | | 8 | <0.5 | 7.8 | 12.4 |
| 2 | 99.0[2] | 1.0 | - | Powder | | 15.9 | - |
| | | | | 4 | <1.0 | 0.8 | 13.4 |
| | | | | 8 | <1.0 | 4.3 | 16.7 |
| 3 | 49.5[1] | 1.0 | 49.5 | Powder | | 58.8 | 7.8 |
| | | | | 4 | <2.0 | 52.8 | 6.2 |
| | | | | 8 | <5.5 | 54.9 | 11.4 |
| 4 | 49.5[2] | 1.0 | 49.5 | Powder | | 44.9 | - |
| | | | | 4 | <30.0 | 57.1 | 2.2 |
| | | | | 8 | <40.0 | 57.1 | 5.0 |

[1] Commercial MCC derived from northern softwoods by the sulfite process, having an alpha cellulose content of about 93% and attrited to a particle size of about 6 to 9 μ m.

[2] Same MCC as (1) but having a particle size about 22 to 25 μ m.

## Claims

1.  A solids dosage form which comprises an active agent and a carrier which disintegrates rapidly in an aqueous medium to release the active agent, characterised in that the carrier comprises:

    a) microcrystalline cellulose;
    b) at least one nitrogen-containing compound which is: urea, ammonium sulfate; ammonium phosphate or a mixture thereof, the nitrogen containing compound being different from the active agent, the ratio by weight of microcrystalline cellulose to nitrogen-containing compound being in the range of 10:1 to 1:10; and
    c) optionally, a minor amount of one or more suspending agents for the microcrystalline cellulose selected from water-soluble hydrocolloids compatible with and effective for assisting in the hydration of the microcrystalline cellulose.

2.  A dosage form according to claim 1, characterised in that the ratio of microcrystalline cellulose to nitrogen containing compound is in the range 2:1 to 1:3.

3.  A dosage form according to any of the preceding claims characterised in that the average particle size of the microcrystalline cellulose is in the range 20 to 90 microns.

4.  A dosage form according to any of the preceding claims characterised in that the suspending agent is present and is a cellulose derivative; a seaweed extract gum; a plant gum; a plant exudate gum; a plant seed extract gum; a microbial fermentation gum; a starch; or a synthetic polymer; or a mixture thereof.

5.  A dosage form according to any of the preceding claims characterised in that the suspending agent is present and is sodium carboxymethyl cellulose.

6.  A dosage form according to any of the preceding claims characterised in that the nitrogen-containing compound is urea.

7.  A dosage form according to any of claims 1 to 5 characterised in that the nitrogen -containing compound is ammonium phosphate.

8.  A dosage form according to any of the claims 1 to 5 characterised in that the nitrogen-containing compound is ammonium sulfate.

9.  A solid dosage form according to any of the preceding claims characterised in that it is produced by compaction of the active agent and the carrier.

10. A solid dosage form according to any of the preceding claims characterised in that the active agent is an agricultural chemical.

11. A solid dosage form according to claim 10 characterised in that active agent is a herbicide or pesticide.

12. A dosage form according to any of the preceding claims characterised in that it comprises an auxiliary disintegrant which is sodium croscarmellose.

13. A method for lowering the disintegration time in water of a compacted composition comprising an active ingredient and microcrystalline cellulose characterised in that a nitrogen containing compound selected from the group comprising urea, ammonium sulphate and ammonium phosphate is incorporated into the composition in an amount such that the ratio of the weight of microcrystalline cellulose to nitrogen containing compound is in the range 10:1 to 1:10.

14. A method according to claim 13 characterised in that the microcrystalline cellulose has a particle size of from 20 to 90 microns.

15. A method according to either of claims 13 to 14 characterised in that the compacted composition is a tablet having the strength of from 2 kg/cm$^2$ to 25 kg/cm$^2$.

16. A method according to any of claims 13 to 15 characterised in that the nitrogen containing compound comprises urea.

17. A method according to any of claims 13 to 15 characterised in that the nitrogen compound comprises ammonium phosphate.

18. A method according to any of claims 13 to 15 characterised in that the nitrogen compound comprises ammonium sulfate.

19. A method according to any of claims 13 to 18 characterised in that the nitrogen containing compound is blended with the microcrystalline cellulose by coprocessing the microcrystalline cellulose and the nitrogen containing compound.

20. A method according to claim 19 characterised in that an aqueous slurry of the microcrystalline cellulose and nitrogen containing compound is formed and the slurry is dried by spray drying.

21. A method according to claim 20 characterised in that urea is present and the ratio of the microcrystalline cellulose to urea is from 2:1 to 1:3.

22. A method according to any of claims 13 to 21 characterised in that the composition comprises from 0.5% to 25%, based on the weight of the microcrystalline cellulose, of a suspending agent selected from water-soluble hydrocolloids compatible with, and effective for assisting in the hydration of the microcrystalline cellulose.

23. A method according to claim 22 characterised in that the suspending agent comprises sodium carboxymethycellulose.

24. A method according to any of claims 13 to 23 characterised in that the active agent is an agricultural chemical.

25. A method according to any of claims 13 to 24 characterised in that the composition comprises an auxiliary disintegrant which is croscarmellose.

**Patentansprüche**

1. Feste Dosierungsform, welche ein aktives Mittel und einen Träger umfaßt, welcher schnell in einem wäßrigen Medium zerfällt, um des aktive Mittel freizusetzen, dadurch gekennzeichnet, daß der Träger umfaßt.

  a) mikrokristalline Zellulose:

  b) mindestens eine Stickstoff-enthaltende Verbindung, welche ist Harnstoff, Ammoniumsulfat, Ammoniumphosphat oder eine Mischung davon, wobei die Stickstoff-enthaltende Verbindung sich von dem Mittel unterschiedet, wobei das Gewichtsverhältnis von mikrokristalliner Zellulose zu Stickstoff-enthaltender Verbindung in einem Bereich von 10:1 bis 1:10 ist; und

  c) gegebenenfalls eine geringe Menge eines oder mehrerer Suspensionsmittel für die mikrokristalline Zellulose, ausgewählt aus wasserlöslichen Hydrokolloiden, welche vereinbar sind mit und wirksam sind zum Unterstützen der Hydratation der mikrokristallinen Zellulose.

2. Dosierungsform nach Anspruch 1, dadurch gekennzeichnet, daß das Verhältnis von mikrokristalliner Zellulose zu Stickstoff-enthaltender Verbindung in einem Bereich von 2:1 bis 1:3 ist.

3. Dosierungsform nach mindestens einem der vorhergehenden Ansprüche, dadurch gekennzeichnet, daß die durchschnittliche Partikelgröße der mikrokritalllinen Zellulose in einem Bereich von 20 bis 90 Micron ist.

4. Dosierungsform nach mindestens einem der vorhergehenden Ansprüche, dadurch gekenzeichnet, daß das Suspensionsmittel anwesend ist und ein Zellulosederivat, ein Seetangextraktharz ein Pflanzenharz, ein Pflanzenausscheldungsproduktharz, ein Pflanzensamenextraktharz, ein mikrobielles Fermentationsharz (engl. microbial fermentation gum), eine Stärke oder ein synthetisches Polymer oder eine Mischung davon ist.

5. Dosierungsform noch mindestens einem der vorhergehenden Ansprüche, dadurch gekennzeichnet, daß das Suspensionsmittel anwesend Ist und Natrimcarboxymethytzellulose ist.

6. Doslerungsform nach mindestens einem der vorhergehenden Ansprüche, dadurch gekennzeichnet, daß die Stickstoff-enthaltende Verbindung Harnstoff ist.

7. Doslerungsform nach mindestens einem der Ansprüche 1 bis 5, dadurch gekennzeichnet daß die Stickstoff-enthaltende Verbindung Ammoniumphosphat ist

8. Dosierungsform nach mindestens einem der Ansprüche 1 bis 5, dadurch gekennzeichnet, daß die Stickstoff-enthaltende Verbindung Ammoniumsulfat ist.

9. Feste Dosierungsform nach mindestens einem der vorgehenden Ansprüche, dadurch gekennzeichnet. daß sie durch Verdichten des aktiven Mittels und des Trägers hergestellt wird.

10. Feste Dosierungsform nach mindestens einem der vorhergehenden Ansprüche, dadurch gekennzeichnet, daß das aktive Mittel eine Agrochemikralle ist.

11. Feste Dosierungsform nach Anspruch 10, dadurch gekennzeichnet. daß des aktive Mittel ein Herbizid oder Pestizid ist.

12. Dosierungsform nach mindestens einem der vorhergehenden Ansprüche, dadurch gekennzeicnnet, daß sie ein Hilfszersetzungsmittel, welches Natriumcroscarmellose ist, umfaßt

13. Verfahren zum Senken der Zersetzungszeit einer verdichten Zusammensetzung in Wasser, umfassend einen aktiven inhaltsstoff und mikrokristalline Zellulose, dadurch gekennzeichnet, daß eine Stickstoff-enthaltende Verbindung, ausgewählt aus der Gruppe, umfassend Harnstoff, Ammoniumsulfat und Ammoniumphosphat, in die Zussammensetzung einer Menge aufgenommen wird, so daß das Gewichtsverhaltnis von mikrokristalliner Zellulose zu Stickstoff-enthaltender Verbindung in dem Bereich 10:1 bis 1:10 ist.

14. Verfahren nach Anspruch 13, dadurch gekennzeichnet, daß die mikrokristalline Zellulose eine Partikelgröße von 20 bis 90 Micron hat.

15. Verfahren nach einem der Ansprüche 13 bis 14, dadurch gekennzeichnet daß die verdichtete Zusammensetzung eine Tablette ist, die eine Zugfestigkeit von 2 kg/cm$^2$ bis 25 kg/cm$^2$ hat.

16. Verfahren nach mindestens einem der Ansprüche 13 bis 15, dadurch gekennzeichnet, daß die Stickstoff-enthaltende Verbindung Harnstoff umfaßt.

17. Verfahren nach mindestens einem der Ansprüche 13 bis 15, dadurch gekennzeichnet, daß die Stickstoffverbindung Ammoniumphosphat umfaßt.

18. Verfahren nach mindestens einem der Ansprüche 13 bis 15, dadurch gekennzeichnet, daß die Stickstoffvorbindung Ammoniumsulfat umfaßt.

19. Verfahren nach mindestens einem der Ansprüche 13 bis 18, dadurch gekennzeichnet, daß die Stickstoff-enthaltende Verbindung mit der mikrokristallinen Zellulose durch gemelnsames Verarbeiten der mikrokristallinen Zellulose und der Stickstoff-enthaltenden Verbindung vermischt wird.

20. verfahren nach Anspruch 19, dadurch gekennzeichnet, daß eine wäßrige Aufschlämmung der mikrokristallinen Zellulose und Stickstoff-enthaltenden Verbindung gebildet wird und die Aufschlämmung durch Sprühtrocknung getrocknet wird.

21. Verfahren nach Anspruch 20, dadurch gekennzeichnet, daß Harnstoff anwesend ist und das Verhaltnis der mikrokristallinen Zellulose zu Harnstoff von 2:1 bis 1:3 ist.

22. Verfahren nach mindestens einem der Ansprüche 13 bis 21, dadurch gekennzeichnet, daß die Verbindung von 0.5% bis 25% eines Suspensionsmittels, basierend auf dem Gewicht der mikrokristallinen Zellulose, umfaßt, ausgewählt aus wasserlöslichen Hydrokolloiden, die vereinbar sind mit und wirksam sind zum Unterstützen der Hydratation der mikrokristallinen Zellulose.

23. Vefahren nach Anspruch 22, dadurch gekennzeichnet, daß das Suspensionsmittel Natriumcarboxymethylzellulose umfaßt.

24. Verfahren nach mindestens einem der Ansprüche 13 bis 23, dadurch gekennzeichnet, daß das aktive Mittel eine Agrochemikalle ist.

25. Verfahren nach mindestens einem der Ansprüche 13 bis 24, dadurch gekennzeichnet, daß die Zusammensetzung ein Hilfazersetzungsmittel umfaßt, welches Croscarmellose ist

## Revendications

1. Forme de dosage solide qui comprend un agent actif et un support qui se désagrège rapidement dans un milieu aqueux pour libérer l'agent actif, caractérisée en ce que le support comprend :

   a) de la cellulose microcristalline;
   b) au moins un composé contenant de l'azote qui est de l'urée, du sulfate d'ammonium, du phosphate d'ammonium ou un mélange de ceux-ci, le composé contenant de l'azote étant différent de l'agent actif, le rapport en poids de la cellulose microcristalline au composé contenant de l'azote se situant dans la gamme de 10/1 à 1/10; et
   c) éventuellement, une petite quantité d'un ou plusieurs agents de mise en suspension pour la cellulose microcristalline choisis parmi les hydrocolloïdes solubles dans l'eau compatibles avec la cellulose microcristalline et efficaces pour faciliter l'hydratation de celle-ci.

2. Forme de dosage suivant la revendication 1, caractérisée en ce que le rapport de la cellulose microcristalline au composé contenant de l'azote se situe dans la gamme de 2/1 à 1/3.

3. Forme de dosage suivant l'une quelconque des revendications précédentes, caractérisée en ce que la taille de particule moyenne de la cellulose microcristalline se situe dans la gamme de 20 à 90 microns.

4. Forme de dosage suivant l'une quelconque des revendications précédentes, caractérisée en ce que l'agent de mise en suspension est présent et est un dérivé cellulosique, une gomme d'extrait d'algue, une gomme végétale, une gomme d'exsudat végétal, une gomme d'extrait de semences végétales, une gomme de fermentation microbienne, un amidon, un polymère synthétique ou un mélange de ceux-ci.

5. Forme de dosage suivant l'une quelconque des revendications précédentes, caractérisée en ce que l'agent de mise en suspension est présent et est la carboxyméthyl cellulose de sodium.

6. Forme de dosage suivant l'une quelconque des revendications précédentes, caractérisée en ce que le composé contenant de l'azote est de l'urée.

7. Forme de dosage suivant l'une quelconque des revendications 1 à 5, caractérisée en ce que le composé contenant de l'azote est du phosphate d'ammonium.

8. Forme de dosage suivant l'une quelconque des revendications 1 à 5, caractérisée en ce que le composé contenant de l'azote est du sulfate d'ammonium.

9. Forme de dosage solide suivant l'une quelconque des revendications précédentes, caractérisée en ce qu'elle est produite par compaction de l'agent actif et du support.

10. Forme de dosage solide suivant l'une quelconque des revendications précédentes, caractérisée en ce que l'agent actif est un composé chimique agricole.

11. Forme de dosage solide suivant la revendication 10, caractérisée en ce que l'agent actif est un herbicide ou pesticide.

**12.** Forme de dosage suivant l'une quelconque des revendications précédentes, caractérisée en ce qu'elle comprend un agent de désagrégation auxiliaire qui est de la croscarmellose de sodium.

**13.** Procédé pour abaisser le temps de désagrégation dans l'eau d'une composition compactée comprenant un ingrédient actif et de la cellulose microcristalline, caractérisé en ce qu'un composé contenant de l'azote choisi dans le groupe comprenant l'urée, le sulfate d'ammonium et le phosphate d'ammonium est incorporé dans la composition en une quantité telle que le rapport en poids de cellulose microcristalline au composé contenant de l'azote se situe dans la gamme de 10/1 à 1/10.

**14.** Procédé suivant la revendication 13, caractérisé en ce que la cellulose microcristalline a une taille de particule de 20 à 90 microns.

**15.** Procédé suivant l'une ou l'autre des revendications 13 et 14, caractérisé en ce que la composition compactée est un comprimé ayant une résistance de 2 kg/cm$^2$ à 25 kg/cm$^2$.

**16.** Procédé suivant l'une quelconque des revendications 13 à 15, caractérisé en ce que le composé contenant de l'azote comprend de l'urée.

**17.** Procédé suivant l'une quelconque des revendications 13 à 15, caractérisé en ce que le composé azoté comprend du phosphate d'ammonium.

**18.** Procédé suivant l'une quelconque des revendications 13 à 15, caractérisé en ce que le composé azoté comprend du sulfate d'ammonium.

**19.** Procédé suivant l'une quelconque des revendications 13 à 18, caractérisé en ce que le composé contenant de l'azote est mélangé à la cellulose microcristalline en cotraitant la cellulose microcristalline et le composé contenant de l'azote.

**20.** Procédé suivant la revendication 19, caractérisé en ce qu'une suspension aqueuse de la cellulose microcristalline et du composé contenant de l'azote est formée et la suspension est séchée par séchage par pulvérisation.

**21.** Procédé suivant la revendication 20, caractérisé en ce que de l'urée est présente et le rapport de la cellulose microcristalline à l'urée est de 2/1 à 1/3.

**22.** Procédé suivant l'une quelconque des revendications 13 à 21, caractérisé en ce que la composition comprend de 0,5% à 25 %, par rapport au poids de la cellulose microcristalline, d'un agent de mise en suspension choisi parmi les hydrocolloïdes solubles dans l'eau compatibles avec la cellulose microcristalline et efficaces pour faciliter l'hydratation de celle-ci.

**23.** Procédé suivant la revendication 22, caractérisé en ce que l'agent de mise en suspension comprend de la carhoxyméthylcellulose de sodium.

**24.** Procédé suivant l'une quelconque des revendications 13 à 23, caractérisé en ce que l'agent actif est un composé chimique agricole.

**25.** Procédé suivant l'une quelconque des revendications 13 à 24, caractérisé en ce que la composition comprend un agent de désagrégation auxiliaire qui est de la croscarmellose.